Europäisches Patentamt

European Patent Office

Office européen des brevets

(19)

(11) **EP 1 146 839 B1**

(12) **EUROPEAN PATENT SPECIFICATION**

(45) Date of publication and mention
of the grant of the patent:
**07.04.2004 Bulletin 2004/15**

(51) Int Cl.⁷: $A61F\ 13/15$

(86) International application number:
**PCT/US1999/002176**

(21) Application number: **99906700.2**

(22) Date of filing: **02.02.1999**

(87) International publication number:
**WO 2000/045762 (10.08.2000 Gazette 2000/32)**

(54) **DISPOSABLE DIAPER**

WEGWERFWINDEL

COUCHE-CULOTTE JETABLE

(84) Designated Contracting States:
**AT BE CH CY DE DK ES FI FR GB GR IE IT LI LU
NL PT SE**

(43) Date of publication of application:
**24.10.2001 Bulletin 2001/43**

(73) Proprietor: **THE PROCTER & GAMBLE COMPANY
Cincinnati, Ohio 45202 (US)**

(72) Inventors:
• **KAWAKAMI, Yoshihisa
Higashinada-ku Kobe 658-0016 (JP)**

• **UEDA, Kimio
Higashinada-ku Kobe 658-0053 (JP)**
• **GRAY, Michael Vincent
Higashinada-ku, Kobe 658-0032 (JP)**

(74) Representative: **McGregor, Judit Ester
Procter & Gamble Service GmbH
Sulzbacher Strasse 40-50
65924 Schwalbach am Taunus (DE)**

(56) References cited:
**WO-A-98/20822       WO-A-98/29247
WO-A-98/29480**

**Description**

FIELD

The present invention relates to disposable diapers (adult and baby) including pull on diapers. More specifically, the present invention relates to disposable diapers which include an outer cover spunbonded nonwoven layer of a polypropylene/polyethylene copolymer.

BACKGROUND

Infants and other incontinent individuals wear disposable garments such as diapers to receive and contain urine and other body exudates. Disposable garments function either or both to contain the discharged materials and to isolate these materials from the body of the wearer and from the wearer's garments and bed clothing. Disposable garments having many different basic designs are known to the art. Examples of such disposable garments include disposable underwears, disposable diapers (adult and baby) including pull-on diapers and training pants, disposable panties for menstrual use, and disposable absorbent pads including sanitary napkins.
It is also known that the exterior of disposable garments is covered with a flexible, liquid and vapor impervious sheet to prevent any absorbed liquid from passing through the disposable garments and soiling adjacent articles such as clothing, bedding and the like. Such a liquid impervious sheet is generally referred to as backsheets, and is often constructed from fluid impervious sheets such as polyethylene films. While such backsheets do prevent liquid from passing through the disposable garments, the use of such a plastic film tends to provide an uncomfortable felling to the wearer and/or user since the touch of plastic film is very different from that of cloth diapers. To address this issue, recent disposable garments tend to employ a "cloth-like" backsheet wherein a nonwoven web is joined to the outer- surface of the plastic film to provide a "cloth-like" looking and touch. While such a "cloth-like" backsheet can provide a soft and smooth touch, it tends not to have enough abrasion resistance against friction which is generally caused by the wearer's movement against adjacent articles such as clothing, bedding and the like. This results in an undesirable fuzz occurrence, i.e., a removal of at least some of the component fibers from the nonwoven web.
WO 98/29247 discloses cloth-like, liquid-impervious, breathable film-nonwoven composite fabrics, comprising at least one nonwoven layer and a microporous film having biological, liquid barrier capabilities for use as, for example, sterilization wrap, surgical draping, surgical gowns, cover garments such as over-suits, and the like.
Based on the foregoing, there is a need for disposable garments which include a "cloth-like" backsheet that can reduce undesirable fuzz occurrence caused by the friction against adjacent articles.

SUMMARY

The present invention is directed to a disposable diaper which comprises a backsheet including an outer cover fabric of a spunbonded nonwoven layer. The spunbonded nonwoven layer includes component fibers formed by a polypropylene/polyethylene copolymer. The component fibers have an average fiber denier of less than 2.5. The spunbonded nonwoven layer has a Coefficient of Friction against Steel (CFS) of less than 0.29. The spunbonded nonwoven layer has a Fuzz Level (FL) of less than 0.15 mg/cm$^2$.
The foregoing answers the need for disposable garments which include a "cloth-like" backsheet that can reduce undesirable fuzz occurrence caused by the friction against adjacent articles.
These and other features, aspects, and advantages of the present invention will become evident to those skilled in the art from reading of the present disclosure.

BRIEF DESCRIPTION OF THE DRAWINGS

While the specification concludes with claims particularly pointing out and distinctly claiming the invention, it is believed that the invention will be better understood from the following description of preferred embodiments which is taken in conjunction with the accompanying drawings and which like designations are used to designate substantially identical elements, and in which:

Fig. 1 is a perspective view of one preferred embodiment of the disposable pull-on garment of the present invention in a typical in use configuration;
Fig. 2 is a simplified plan view of the embodiment shown in Fig. 1 in its flat uncontracted condition showing the body-facing side the garment;
Fig. 3 is a cross-sectional view of a preferred embodiment taken along the section line 3-3 of Fig. 2;
Fig. 4 is a cross-sectional view of an elastic member of a preferred embodiment;

Fig. 5 is a fragmentary enlarged perspective illustration of one preferred example of the elastomeric material layer;

Fig. 6 is a graph showing the tensile property of a sample nonwoven:

Figs. 7A and 7B are schematic diagrams explaining the measurement for the bending property;

Fig. 8 is a graph showing the bending property of a sample nonwoven;

Fig. 9A and 9B are schematic diagrams explaining the measurement for the surface roughness of a sample nonwoven;

Fig. 10A and 10B are schematic diagrams explaining the measurement for the surface friction of a sample nonwoven;

Fig. I 1 shows the conditions of the steel plate used for the surface roughness and friction measurements;

Fig. 12 shows the changes of the friction coefficient along the surface of a sample nonwoven;

Fig. 13 shows the changes of the thickness along the surface of a sample nonwoven;

Fig. 14 is a plane view of a sample nonwoven used for the shearing property measurement;

Fig. 15 is a graph showing the shearing property of a sample nonwoven;

Fig. 16 is a plane view of a sample nonwoven used for the compression property measurement;

Fig. 17 is a graph showing the compression property of a sample nonwoven; and

Fig. 18 is a schematic diagram explaining the fuzz level measurement of a sample nonwoven.

## DETAILED DESCRIPTION

Citation of any reference is not an admission regarding any determination as to its availability as prior art to the claimed invention.

Herein, "comprise" means that other element(s) and step(s) which do not affect the end result can be added. These terms encompass the terms "consisting of" and "consisting essentially of".

Herein, "pull-on garment" refers to articles of wear which have a defined waist opening and a pair of leg openings and which are pulled onto the body of the wearer by inserting the legs into the leg openings and pulling the article up over the waist.

Herein, "disposable" describes garments which are not intended to be laundered or otherwise restored or reused as a garment (i.e., they are intended to be discarded after a single use and, preferably, to be recycled, composted or otherwise disposed of in an environmentally compatible manner).

Herein, "pull-on diaper" refers to pull-on garments generally worn by infants and other incontinent individuals to absorb and contain urine and feces. It should be understood, however, that the present invention is also applicable to other pull-on garments such as training pants, incontinent briefs, feminine hygiene garments or panties. and the like.

Herein, "panel" denotes an area or element of the pull-on garment. (While a panel is typically a distinct area or element, a panel may coincide (functionally correspond) somewhat with an adjacent panel.)

Herein, "layer" does not necessarily limit the element to a single strata of material in that a layer may actually comprise laminates or combinations of sheets or webs of the requisite type of materials.

Herein, "joined" or "joining" encompasses configurations whereby an element is directly secured to another by affixing the element directly to the other element, and configurations whereby the element is indirectly secured to the other element by affixing the element to intermediate member(s) which in turn are affixed to the other element.

Herein, "uncontracted state" is used to describe states of pull-on garments in its unseamed (i.e., seams are removed), flat and relaxed condition wherein all elastic materials used are removed therefrom.

The present invention can be applied to a variety of disposable articles in need of a soft "cloth-like" backsheet that includes a nonwoven layer which can reduce undesirable fuzz occurrence caused by the friction against adjacent articles. Preferred disposable garment include disposable underwears, disposable diapers (adult and baby) including pull-on diapers and training pants, disposable panties for menstrual use, and disposable absorbent pads including sanitary napkins and incontinence devices.

The disposable garment of the present invention includes a backsheet which includes an outer cover of a spunbonded nonwoven layer. The spunbonded nonwoven layer includes component fibers formed by a polypropylene/polyethylene copolymer. In a preferred embodiment, the backsheet further includes a liquid impervious layer joined to the body-facing surface of the outer cover fabric. More preferably, the liquid impervious layer is a microporous polyethylene film joined to the body-facing surface of the outer cover fabric by an adhesive. These and other preferred embodiments of the present invention will be described in detail hereinafter.

By employing the spunbonded nonwoven layer of polypropylene/polyethylene copolymer in the outer cover of the backsheet, the backsheet of the present invention provides enough abrasion resistance against friction which is generally caused by the wearer's movement against adjacent articles such as clothing, bedding and the like. Consequently, the backsheet can reduce undesired fuzz occurrence caused by the friction between the disposable garment and adjacent articles, while providing a soft "cloth-like" looking and a soft and smooth touch.

The spunbonded nonwoven layer of the present invention can be formed by any spunbonded nonwoven process known in the art, for example, U.S. Patent No. 3,692,618 issued to Dorschner et al. on September 19, 1972; U.S. Patent No. 3,338,992 issued to Kinney on August 29, 1967; U.S. Patent No. 3,341,394 issued to Kinney on September 12, 1967; U.S. Patent No. 3,502,538 issued to Petersen on March 24, 1970; U.S. Patent No. 3,502,763 issued to Hartmann on March 24, 1970; U.S. Patent No. 3,909,009 issued to Cvetko et al. on September 30, 1975; U.S. Patent No. 3,542,615 issued to Dobo et al. on November 24, 1970; and U.S. Patent No. 4,340,563 issued to Appel et al. on July 20, 1982.

The polypropylene/polyethylene copolymer which forms the component fibers is produced by copolymerizing from 0.5% to 20% by weight of polyethylene in the backbone of a polypropylene. Preferably, the polypropylene copolymer is a block copolymer produced by copolymerizing the polyethylene by block in the backbone of polypropylene. More preferably, the polypropylene/polyethylene copolymer is a random copolymer produced by copolymerizing the polyethylene randomly in the backbone of polypropylene.

Preferably, from about 0.5% to about 20%, more preferably from about 5% to about 15% by weight of polyethylene is contained by copolymerization in the backbone of polypropylene. A preferred polypropylene/polyethylene is produced when about 7% by weight of polyethylene is copolymerized with the polypropylene.

The polypropylene/polyethylene copolymer is less crystalline and thus has a broader melting point range than polypropylene itself. Preferably, the polypropylene/polyethylene copolymer has a melting point range between 100 °C and 180 °C, more preferably between 125 °C and 165 °C. The broadened melting point range allows for heat bonding of the component fibers over a wider range of nonwoven formation temperature. Thus, the component fibers can be bonded by a stable nonwoven formation process, whereby the abrasion resistance against friction of the resulting nonwoven web can be improved.

Any type of polyethylene can be employed in the copolymerization. Preferred polyethylene includes a low density polyethylene (LDPE), a high density polyethylene (HDPE) and a linear low density polyethylene (LLDPE). A preferred polyethylene is a LLDPE. In a preferred embodiment, the polyethylene has an $\alpha$ olefin structure such as 1-hexene, 1-butene and 1-octene.

In a preferred embodiment, the spunbonded nonwoven layer has a basis weight of less than about 35 g/m$^2$, and comprises component fibers having an average fiber denier of less than about 2.5. Preferably, for products such as disposable garment and the like, the spunbonded nonwoven layer has a basis weight of from about 15 g/m$^2$ to about 30 g/m$^2$, more preferably from about 17 g/m$^2$ to about 25 g/m$^2$, and an average fiber denier of less than about 1.9, and more preferably less than about 1.5.

In a preferred embodiment, the spunbonded nonwoven layer has an embossment pattern formed by a number of discrete bonding spots or areas. Any embossment process known in the art can be used for forming such an embossment pattern in spunbonded nonwoven layer. For example, a preferred embossment pattern is formed by feeding a spunbonded nonwoven web through two bonding rolls which have a raised pattern such as spots or grids on the surface of the bonding rolls. The bonding rolls are heated to a softening temperature of the material of the component fibers (i.e., the polypropylene/polyethylene copolymer). The spunbonded nonwoven web passes between the heated bonding rolls so that the spunbonded nonwoven web can be compressed and heated by the bonding rolls in accordance with the pattern on the rolls, thereby forming an embossment pattern of discrete bonding spots or areas in the spunbonded nonwoven web. The resulting spunbonded nonwoven web is preferably used as the spunbonded nonwoven layer. As is well known in the art, the embossment holds the component fibers together and imparts an integrity to the nonwoven web or layer by bonding the component fibers within the nonwoven web or layer.

A preferred embossment pattern has lozenge shaped pin bonding areas wherein each pin has an average dimension of about 0.65 x 0.74 mm, a vertical spacing of about 1.23 mm between pins, and a horizontal spacing of about 1.26 mm between pins. Preferably, the embossment pattern has an embossment area (or a bonded area) ratio of less than about 25 %. In a preferred embodiment, the embossment area ratio of the nonwoven web or layer is from about 5% to about 20%, and more preferably from about 10% to about 18%.

The spunbonded nonwoven layer has a Fuzz Level (FL) of less than 0.15 mg/cm$^2$, preferably less, than 0.1 mg/cm$^2$, and more preferably from 0.03 mg/cm$^2$ to 0.09 mg/cm$^2$. The method for measuring the Fuzz Level of nonwoven webs or layers is explained in more detail in the Test Method Section.

The component fibers of the spunbonded nonwoven layer has a Coefficient of Friction against Steel (CFS) of less than 0.29, preferably less than 0.17, and more preferably from 0.1 to 0.15. The measurement of the Coefficient of Friction against Steel is performed according to ASTM (American Standard Test Method) Standard D 1894-63 (Reapproval 1972).

In a preferred embodiment, the spunbonded nonwoven layer has a hand value of Koshi of less than 11, preferably greater than about 5, and more preferably from 6 to 8.5. The method for measuring the hand value of Koshi of nonwoven webs or layers is explained in more detail in the Test Method Section.

In a preferred embodiment, the spunbonded nonwoven layer has a Young's modulus of greater than 4000 gf/inch (1575 gf/cm), preferably greater than 5000 gf/inch (1969 gf/cm), and more preferably from 5000 gf/inch (1969 gf/cm) to 12000 gf/inch (4724 gf/cm).

In a preferred embodiment, the spunbonded nonwoven layer has a stress of less than 1000 gf/inch (394 gf/cm) at peak in the Stress-Strain curve. Preferably, the spunbonded nonwoven layer has a stress of greater than 600 gf/inch (236 gf/cm), more preferably from 700 gf/inch (276 gf/cm) to 1000 gf/inch (394 gf/cm) at peak in the Stress-Strain curve.

A preferred spunbonded nonwoven web which is used for the spunbonded nonwoven layer of polypropylene/polyethylene copolymer is available from Mitsui Chemical Co., Ltd., Tokyo, Japan, under Code No. PC-0220. This spunbonded nonwoven web includes component fibers formed by a random polypropylene/polyethylene copolymer which has 7% by weight of polyethylene (LLPE) in the backbone of polypropylene. This spunbonded nonwoven web has a basis weight of 20 g/m$^2$, and includes component fibers having an average fiber denier of 20. This spunbonded nonwoven web has an average embossment ratio of 18%. The polypropylene/polyethylene copolymer of this spunbonded nonwoven web has a melting point range between 125 °C and 165 °C. This spunbonded nonwoven web has a hand value of Koshi of 9.1.

In a preferred embodiment, the disposable garment of the present invention is a disposable pull-on garment. In the following, such a disposable pull-on garment will be described in detail by referring to drawings.

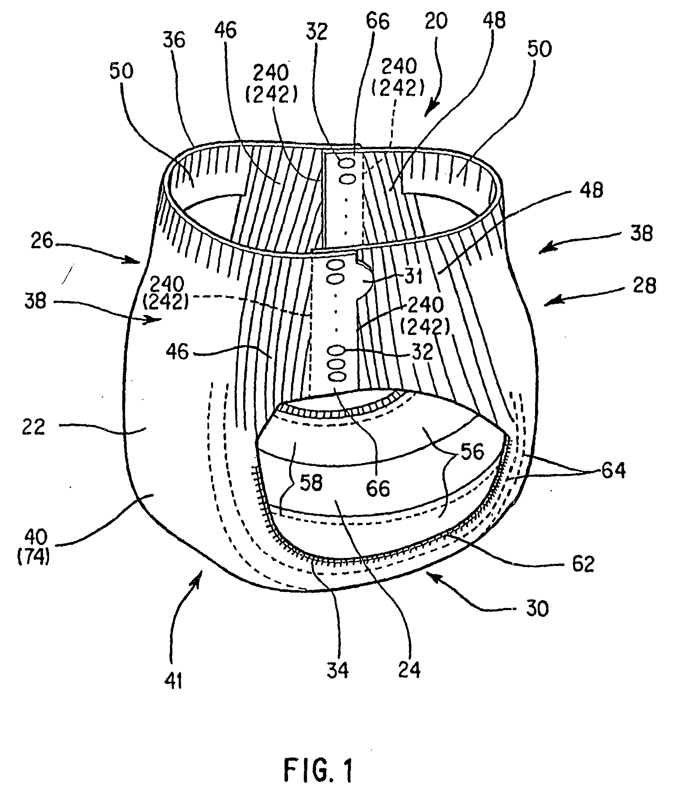

Fig. 1 shows one preferred embodiment of a disposable pull-on garment of the present invention (e.g., a unitary disposable pull-on diaper). Referring to Fig. 1, the disposable pull-on garment 120 has a front region 26; a back region 28 and a crotch region 30 between the front region 26 and the back region 28. A chassis 41 is provided in the front, back and crotch regions 26, 28 and 30. The chassis 41 includes a liquid pervious topsheet 24, a liquid impervious backsheet 22 associated with the topsheet 24, and an absorbent core 25 (not shown in Fig. 1) disposed between the topsheet 24 and the backsheet 22. The chassis 41 has side edges 220 which form edge lines 222 in the front region 26.

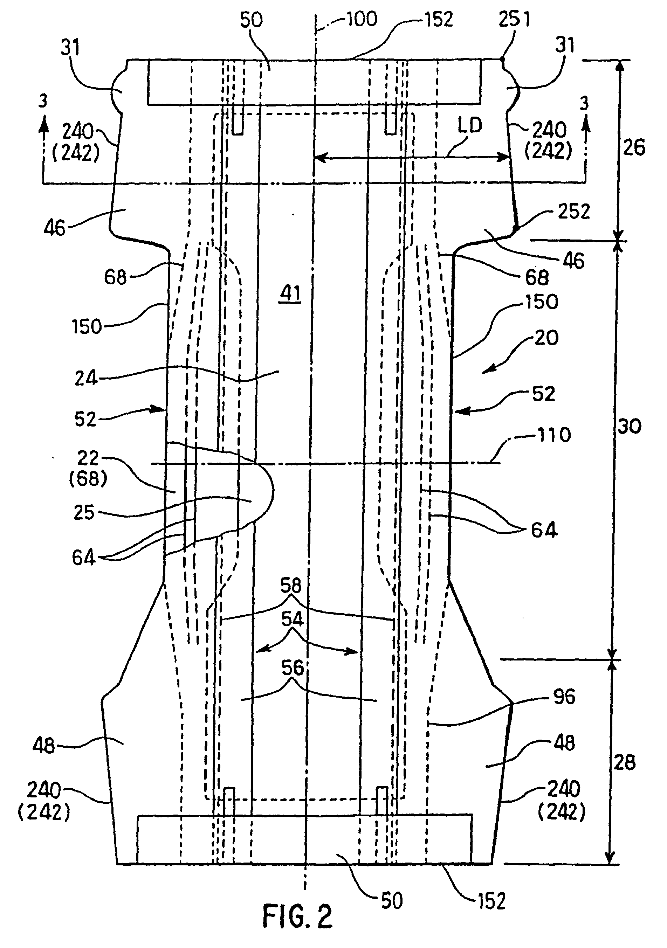

The disposable pull-on garment 20 further includes a pair of front ear panels 46 each extending laterally outward from the corresponding sides of the chassis 41 in the front region 26, and a pair of extensible back ear panels 48 each extending laterally outward from the corresponding sides of the chassis 41 in the back region 28. Each of the ear panels 46 and 48 has an outermost edge 240 which forms an outermost edge line 242. At least one of the outermost edge lines 242 has a nonuniform lateral distance LD from the longitudinal center line 100 (not shown in Fig. 1 but in Fig. 2) in the uncontracted state of the garment 20. The pull-on garment 20 further includes seams 32 each joining the front and back ear panels 46 and 48 along the corresponding edge lines 242 to form the two leg openings 34 and the waist opening 36.

In preferred embodiments, the pull-on garment 20 includes a chassis layer 40 which generally determines the overall shape of the pull-on garment 20. In the embodiment shown in Fig. 1, the chassis layer 40 is an outer cover nonwoven layer 74 which covers all of the outer-facing surface of the pull-on garment 20 to provide the feel and appearance of a cloth garment. Preferably, the outer cover nonwoven layer 74 is a continuous sheet or web formed by the spunbonded nonwoven of polypropylene/polyethylene copolymer of the present invention. The continuous sheet (i.e., the outer cover nonwoven layer 74) defines the front region 26, the back region 28 and the crotch region 30 between the front region 26 and the back region 28. Each of the ear panels 46 and 48 includes a portion of the chassis layer 40. Preferred pull-on garments which includes such a continuous sheet are disclosed in U.S. Patent No. 5,569,234 issued to Buell et al. on October 29, 1996.

In a preferred embodiment, at least one of, more preferably both of, the pairs of the ear panels 46 and 48 are elastically extensible in at least the lateral direction. In alternative embodiments, the ear panels 46 and 48 are elastically extensible both in the lateral and longitudinal directions. Herein, "extensible" refers to materials that are capable of extending in at least one direction to a certain degree without undue rupture. Herein, "elasticity" and "elastically extensible" refer to extensible materials that have the ability to return to approximately their original dimensions after the force that extended the material is removed. Herein, any material or element described as "extensible" may also be elastically extensible unless otherwise provided. The extensible ear panels 46 and 48 provide a more comfortable and contouring fit by initially conformably fitting the pull-on garment to the wearer and sustaining this fit throughout the time of wear well past when the pull-on garment has been loaded with exudates since the ear panels 46 and/or 48 allow the sides of the pull-on garment to expand and contract.

The ear panels 46 and 48 may be formed by unitary elements of the pull-on garment 20 (i.e., they are not separately manipulative elements secured to the pull-on garment 20, but rather are formed from and are extensions of one or more of the various layers of the pull-on garment). In a preferred embodiment, the ear panels 46 and 48 include at least one unitary element or a continuous sheet (e.g. the chassis layer 40) that forms a part of the chassis 41 and continuously extends into the ear panels 46 and 48. Alternatively, the ear panels 46 and 48 may only include discrete members (not shown in Figs.) which do not have any unitary element that also forms a part of the chassis 41. Such an ear panel structure may be formed by joining the discrete members to the corresponding sides of the chassis 41.

In a preferred embodiment, the pull-on garment 20 further includes seam panels 66 each extending laterally outward from each of the ear panels 46 and 48; and tear open tabs 31 each extending laterally outward from the seam panel 66. In a preferred embodiment, each of the seam panels 66 is an extension of the corresponding ear panels 46 and 48, or at least one of the component elements used therein (e.g., the chassis layer 40), or any other combination of the elements. More preferably, each of the tear open tabs 31 is also an extension of the corresponding seam panel 66 or at least one of its component elements used therein (e.g., the chassis layer 40), or any other combination of its elements.

In a preferred embodiment, the corresponding edge portions of the ear panels 46 and 48 are joined through the seam panels 66 in an overlapping manner to make an overlapped seam structure as shown in Fig. 1. Alternatively, the front and ear panels 46 and 48 can be seamed in a butt seam manner (not shown in Figs.). The bonding of the seams 32 can be performed by any suitable means known in the art appropriate for the specific materials employed in the chassis 41 and/or the ear panels 46 and 48. Thus, sonic sealing, heat sealing, pressure bonding, adhesive or cohesive bonding, sewing, autogeneous bonding, and the like may be appropriate techniques. Preferably, the seam panels 66 are joined by a predetermined pattern of heat/pressure or ultrasonic welds which withstands the forces and stresses generated on the garment 20 during wear.

A continuous belt 38 is formed by the ear panels 46 and 48, and a part of the chassis 41 about the waist opening 36 as shown in Fig. 1. Preferably, elasticized waist bands 50 are provided in both the front region 26 and the back region 28. The continuous belt 38 acts to dynamically create fitment forces in the pull-on garment 20 when positioned on the wearer, to maintain the pull-on garment 20 on the wearer even when loaded with body exudates thus keeping the absorbent core 25 (not shown in Fig. 1) in close proximity to the wearer, and to distribute the forces dynamically generated during wear about the waist thereby providing supplemental support for the absorbent core 25 without binding or bunching the absorbent core 25.

Fig. 2 is a partially cut-away plan view of the pull-on garment 20 of Fig. 1 in its uncontracted state (except in the ear panels 46 and 48 which are left in their relaxed condition) with the topsheet 24 facing the viewer, prior to the ear panels 46 and 48 being joined together by the seams 32. The pull-on garment 20 has the front region 26, the back region 28 opposed to the front region 26, the crotch region 30 positioned between the front region 26 and the back region 28, and a periphery which is defined by the outer perimeter or edges of the pull-on garment 20 in which the side edges are designated 115 and 240, and the end edges or waist edges are designated 152. The topsheet 24 has the body-facing surface of the pull-on garment 20 which is positioned adjacent to the wearer's body during use. The backsheet 22 has the outer-facing surface of the pull-on garment 20 which is positioned away from the wearer's body. The pull-on garment 20 includes the chassis 41 including the liquid pervious topsheet 24, the liquid impervious backsheet 22 associated with the topsheet 24, and the absorbent core 25 positioned between the topsheet 24 and the backsheet 22. The garment 20 further includes the front and back ear panels 46 and 48 extending laterally outward from the chassis 41, the elasticized leg cuffs 52, and the elasticized waistbands 50. The topsheet 24 and the backsheet 22 have length and width dimensions generally larger than those of the absorbent core 25. The topsheet 24 and the backsheet 22 extend beyond the edges of the absorbent core 25 to thereby form the side edges 115 and the waist edges 152 of the garment 20. The liquid impervious backsheet 22 preferably includes a liquid impervious plastic film 68.

The pull-on garment 20 also has two centerlines, a longitudinal centerline 100 and a transverse centerline 110. Herein, "longitudinal" refers to a line, axis, or direction in the plane of the pull-on garment 20 that is generally aligned with (e.g. approximately parallel with) a vertical plane which bisects a standing wearer into left and right halves when the pull-on garment 20 is worn. Herein, "transverse" and "lateral" are interchangeable and refer to a line, axis or direction which lies within the plane of the pull-on garment that is generally perpendicular to the longitudinal direction (which divides the wearer into front and back body halves). The pull-on garment 20 and component materials thereof also have a body-facing surface which faces the skin of wearer in use and an outer-facing surface which is the opposite surface to the body-facing surface.

While the topsheet 24, the backsheet 22, and the absorbent core 25 may be assembled in a variety of well known configurations, exemplary chassis configurations are described generally in U.S. Patent 3,860,003 entitled "Contractible Side Portions for Disposable Diaper" which issued to Kenneth B. Buell on January 14, 1975; and U.S. Patent 5,151,092 entitled "Absorbent Article With Dynamic Elastic Waist Feature Having A Predisposed Resilient Flexural Hinge" which issued to Kenneth B. Buell et al., on September 29, 1992.

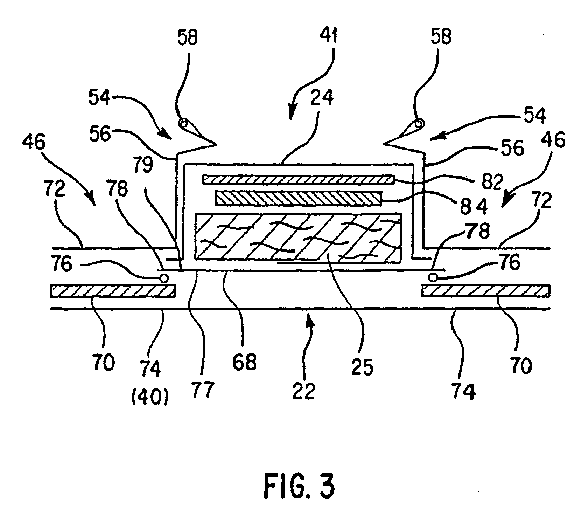

Fig. 3 is a cross-sectional view of a preferred embodiment taken along the section line 3-3 of Fig. 2. The pull-on garment 20 includes the chassis 41 including the liquid pervious topsheet 24, the liquid impervious backsheet 22 associated with the topsheet 24, and the absorbent core 25 positioned between the topsheet 24 and the backsheet 22. The pull-on garment further includes the front ear panels 46 each extending laterally outward from the chassis 41, and an inner barrier cuffs 54. Although Fig. 3 depicts only the structure of the front ear panel 46 and the chassis 41 in the front region 26, preferably a similar structure is also provided in the back region 28. In a preferred embodiment, each of the front ear panels 46 is formed by a lamination of an extended part 72 of the barrier flap 56, an elastic member 70 and the outer cover nonwoven layer 74. The elastic member 70 includes a plane elastomeric material layer 124 (not shown in Fig. 3). Herein, "plane elastomeric material" refers to elastomeric materials which continuously extend in two dimensional directions. Preferred plane elastomeric materials include a scrim, a perforated (or apertures formed) film, an elastomeric woven or nonwoven, an elastomeric foam and the like. In a preferred embodiment, the plane elastomeric material layer 124 includes at least a portion that has a nonuniform lateral width.

The absorbent core 25 can be any absorbent member which is generally compressible, conformable, non-irritating to the wearer's skin, and capable of absorbing and retaining liquids such as urine and other certain body exudates. The absorbent core 25 may be manufactured in a wide variety of sizes and shapes (e.g., rectangular, hourglass, "T"-shaped, asymmetric, etc.) and from a wide variety of liquid-absorbent materials commonly used in disposable pull-on garments and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials.

The configuration and construction of the absorbent core 25 may vary (e.g., the absorbent core 25 may have varying caliper zones, a hydrophilic gradient, a superabsorbent gradient, or lower average density and lower average basis weight acquisition zones; or may include one or more layers or structures). Further, the size and absorbent capacity of the absorbent core 25 may also be varied to accommodate wearers ranging from infants through adults. However, the total absorbent capacity of the absorbent core 25 should be compatible with the design loading and the intended use of the garment 20.

A preferred embodiment of the garment 20 has an asymmetric, modified hourglass-shaped absorbent core 25 having ears in the front and back waist regions 26 and 28. Other exemplary absorbent structures for use as the absorbent core 25 that have achieved wide acceptance and commercial success are described in U.S. Patent No. 4,610,678 entitled "High-Density Absorbent Structures" issued to Weisman et al. on September 9, 1986; U.S. Patent No. 4,673,402 entitled "Absorbent Articles With Dual-Layered Cores" issued to Weisman et al. on June 16, 1987; U. S. Patent No. 4,888,231 entitled "Absorbent Core Having A Dusting Layer" issued to Angstadt on December 19, 1989; and U.S. Patent No. 4,834,735, entitled "High Density Absorbent Members Having Lower Density and Lower Basis Weight Acquisition Zones", issued to Alemany et al. on May 30, 1989.

The chassis 41 may further include an acquisition/distribution core 84 of chemically stiffened fibers positioned over the absorbent core 25, thereby forming a dual core system. In a preferred embodiment, the fibers are hydrophilic chemically stiffened cellulosic fibers. Herein, "chemically stiffened fibers" means any fibers which have been stiffened by chemical means to increase stiffness of the fibers under both dry and aqueous conditions. Such means include the addition of chemical stiffening agents which, for example, coat and/or impregnate the fibers. Such means also include the stiffening of the fibers by altering the chemical structure of the fibers themselves, e.g., by cross-linking polymer chains.

The fibers utilized in the acquisition/distribution core 84 can also be stiffened by means of chemical reaction. For example, crosslinking agents can be applied to the fibers which, subsequent to application, are caused to chemically form intrafiber crosslink bonds. These crosslink bonds can increase stiffness of the fibers. Whereas the utilization of intrafiber crosslink bonds to chemically stiffen the fibers is preferred, it is not meant to exclude other types of reactions for chemical stiffening of the fibers.

In the more preferred stiffened fibers, chemical processing includes intrafiber crosslinking with crosslinking agents while such fibers are in a relatively dehydrated, defibrated (i.e. individualized), twisted, curled condition. Suitable chemical stiffening agents include monomeric crosslinking agents including, but not limited to, $C_2$-$C_8$ dialdehydes and $C_2$-$C_8$ monoaldehydes having an acid functionality can be employed to form the cosslinking solution. These compounds are capable of reacting with at least two hydroxyl groups in a single cellulose chain or on proximately located cellulose chains in a single fiber. Such crosslinking agents contemplated for use in preparing the stiffened cellulose fibers include, but are not limited to, glutaraldehyde, glyoxal, formaldehyde, and glyoxylic acid. Other suitable stiffening agents are polycarboxylates, such as citric acid. The polycarboxylic stiffening agents and a process for making stiffened fibers from them are described in U.S. Patent No. 5,190,563, entitled "Process for Preparing Individualized, Polycarboxylic Acid crosslinked Fibers" issued to Herron, on March 2, 1993. The effect of crosslinking under these conditions is to form fibers which are stiffened and which tend to retain their twisted, curled configuration during use in the absorbent articles herein. Such fibers, and processes for making them are cited in the above incorporated patents.

Preferred dual core systems are disclosed in U.S. Patent No. 5,234,423, entitled "Absorbent Article With Elastic Waist Feature and Enhanced Absorbency" issued to Alemany et al., on August 10, 1993; and in U.S. Patent No. 5,147,345, entitled "High Efficiency Absorbent Articles For Incontinence Management" issued to Young, LaVon and Taylor on September 15, 1992. In a preferred embodiment, the acquisition/distribution core 84 includes chemically treated stiffened cellulosic fiber material, available from Weyerhaeuser Co. (U.S.A.) under the trade designation of "CMC". Preferably, the acquisition/distribution core 84 has a basis weight of from about 40 $g/m^2$ to about 400 $g/m^2$, more preferably from about 75 $g/m^2$ to about 300 $g/m^2$.

More preferably, the chassis 22 further includes an acquisition/distribution layer 82 between the topsheet 24 and the acquisition/distribution core 84 as shown in Fig. 3. The acquisition/distribution layer 82 is provided to help reduce the tendency for surface wetness of the topsheet 24. The acquisition/distribution layer 82 preferably includes carded, resin bonded hiloft nonwoven materials such as, for example, available as Code No. FT-6860 from Polymer Group, Inc., North America (Landisiville, New Jersey, U.S.A.), which is made of polyethylene telephthalate fibers of 6 dtex, and has a basis weight of about 43 g/m$^2$. A preferable example for the acquisition/distribution layer 82 and the acquisition/distribution core 84 is disclosed in EP 0797968A1 (Kurt et al.) published on October 1, 1997.

The topsheet 24 is preferably compliant, soft feeling, and non-irritating to the wearer's skin. Further, the topsheet 24 is liquid pervious permitting liquids (e.g., urine) to readily penetrate through its thickness. A suitable topsheet 24 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be included of natural fibers (e.g., wood or cotton fibers), synthetic fibers (e.g., polymeric fibers such as polyester, polypropylene, or polyethylene fibers) or from a combination of natural and synthetic fibers. The topsheet 24 is preferably made of a hydrophobic material to isolate the wearer's skin from liquids which have passed through the topsheet 24 and are contained in the absorbent core 25 (i.e., to prevent rewet). If the topsheet 24 is made of a hydrophobic material, at least the upper surface of the topsheet 24 is treated to be hydrophilic so that liquids will transfer through the topsheet more rapidly. This diminishes the likelihood that body exudates will flow off the topsheet 24 rather than being drawn through the topsheet 24 and being absorbed by the absorbent core 25. The topsheet 24 can be rendered hydrophilic by treating it with a surfactant. Suitable methods for treating the topsheet 24 with a surfactant include spraying the topsheet 24 material with the surfactant and immersing the material into the surfactant. A more detailed discussion of such a treatment and hydrophilicity is contained in U.S. Patent No. 4,988,344 entitled "Absorbent Articles with Multiple Layer Absorbent Layers" issued to Reising, et al. on January 29, 1991 and U.S. Patent No. 4,988,345 entitled "Absorbent Articles with Rapid Acquiring Absorbent Cores" issued to Reising on January 29, 1991.

In a preferred embodiment, the topsheet 24 is a nonwoven web that can provide reduced tendency for surface wetness; and consequently facilitate maintaining urine absorbed by the core 25 away from the user's skin, after wetting. One of the preferred topsheet materials is a thermobonded carded web which is available as Code No. P-8 from Fiberweb North America, Inc. (Simpsonville, South Carolina, U.S.A.). Another preferred topsheet material is available as Code No. S-2355 from Havix Co., Japan. This material is a bi-layer composite material, and made of two kinds of synthetic surfactant treated bicomponent fibers by using carding and air-through technologies. Yet another preferred topsheet material is a thermobonded carded web which is available as Code No. Profleece Style 040018007 from Amoco Fabrics, Inc. (Gronau, Germany).

Another preferred topsheet 24 includes an apertured formed film. Apertured formed films are preferred for the topsheet 24 because they are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Patent No. 3,929,135, entitled "Absorptive Structures Having Tapered Capillaries", issued to Thompson on December 30, 1975; U.S. Patent No. 4,324,246 entitled "Disposable Absorbent Article Having A Stain Resistant Topsheet", issued to Mullane, et al. on April 13, 1982; U.S. Patent No. 4,342,314 entitled "Resilient Plastic Web Exhibiting Fiber-Like Properties", issued to Radel. et al. on August 3, 1982; U.S. Patent No. 4,463,045 entitled "Macroscopically Expanded Three-Dimensional Plastic Web Exhibiting Non-Glossy Visible Surface and Cloth-Like Tactile Impression", issued to Ahr et al. on July 31, 1984; and U.S. 5,006,394 "Multilayer Polymeric Film" issued to Baird on April 9, 1991.

In a preferred embodiment, the backsheet 22 includes the liquid impervious film 68 as shown in, for example, Fig. 3. Preferably, the liquid impervious film 68 longitudinally extends in the front, back and crotch regions 26, 28 and 30. More preferably, the liquid impervious film 68 does not laterally extend into the at least one of the ear panels 46 or 48. The liquid impervious film 68 has a body-facing surface 79 and an outer-facing surface 77 opposing the body-facing surface 79. The liquid impervious film 68 is impervious to liquids (e.g., urine) and is preferably manufactured from a thin plastic film. However, more preferably the plastic film permits vapors to escape from the garment 20. In a preferred embodiment, a microporous polyethylene film is used for the liquid impervious film 68. A suitable microporous polyethylene film is manufactured by Mitsui Toatsu Chemicals, Inc., Nagoya, Japan and marketed in the trade as PG-P. In a preferred embodiment, a disposable tape (not shown in Figs.) is additionally joined to the outer surface of the backsheet 22 to provide a convenient disposal after soiling. A preferred disposal tape (or device) for pull-on garments is disclosed in International Publication No. WO 94/09736 (Rollag et al.) published on May 11, 1994.

A suitable material for the liquid impervious film 68 is a thermoplastic film having a thickness of from about 0.012 mm (0.5 mil) to about 0.051 mm (2.0 mils), preferably including polyethylene or polypropylene. Preferably, the liquid impervious film has a basis weight of from about 5 g/m$^2$ to about 35 g/m$^2$. However, it should be noted that other flexible liquid impervious materials may be used.

The backsheet 22 further includes the outer cover nonwoven layer 74 (i.e., the chassis layer 40) which is joined with the outer-facing surface 77 of the liquid impervious film 68 to form a laminate. The outer cover nonwoven layer 74 is formed by the spunbonded nonwoven layer of the polypropylene/polyethylene copolymer of the present invention. The outer cover nonwoven layer 74 preferably covers all of the outer-facing surface of the pull-on garment 20 to provide the feel and appearance of a cloth garment. The outer cover nonwoven layer 74 may be joined to the liquid impervious film 68 by any suitable attachment means known in the art. For example, the outer cover nonwoven layer 74 may be secured to the liquid impervious film 68 by a uniform continuous layer of adhesive, a patterned layer of adhesive, or an array of separate lines, spirals, or spots of adhesive. Suitable adhesives include a hotmelt adhesive obtainable from Nitta Findley Co., Ltd., Osaka, Japan as H-2128, and a hotmelt adhesive obtainable from H.B. Fuller Japan Co., Ltd., Osaka, Japan as JM-6064.

The outer cover nonwoven layer 74 is a spunbonded nonwoven web of polypropylene/polyethylene copolymer, for example, obtainable from Mitsui Chemical Co., Ltd., Tokyo, Japan, under Code No. PC-0220. The outer cover nonwoven layer 74 is formed from component fibers of the polypropylene/polyethylene copolymer.

The backsheet 22 is positioned adjacent the outer-facing surface of the absorbent core 25 and is preferably joined thereto by any suitable attachment means known in the art. Specifically, the body-facing surface 79 of the liquid impervious film 68 may be secured to the absorbent core 25 by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota, U.S.A., and marketed as HL-1358J. An example of a suitable attachment means including an open pattern network of filaments of adhesive is disclosed in U.S. Patent No. 4,573,986 entitled "Disposable Waste-Containment Garment", which issued to Minetola et al. on March 4, 1986. Another suitable attachment means including several lines of adhesive filaments swirled into a spiral pattern is illustrated by the apparatus and methods shown in U.S. Patent No. 3,911,173 issued to Sprague, Jr. on October 7, 1975; U.S. Patent No. 4,785,996 issued to Ziecker, et al. on November 22, 1978; and U.S. Patent No. 4,842,666 issued to Werenicz on June 27, 1989. Alternatively, the attachment means may include heat bonds, pressure bonds, ultrasonic bonds. dynamic mechanical bonds, or any other suitable attachment means or combinations of these attachment means as are known in the art.

In an alternative embodiment, the absorbent core 25 is not joined to the backsheet 22, and/or the topsheet 24 in order to provide greater extensibility in the front region 26 and the back region 28.

The pull-on garment 20 preferably further includes elasticized leg cuffs 52 for providing improved containment of liquids and other body exudates. The elasticized leg cuffs 52 may include several different embodiments for reducing the leakage of body exudates in the leg regions. (The leg cuffs can be and are sometimes also referred to as leg bands, side flaps, barrier cuffs, elastic cuffs or gasketing cuffs.) U.S. Patent 3,860,003 entitled "Contractable Side Portions for Disposable Diaper" issued to Buell on January 14, 1975, describes a disposable diaper which provides a contractible leg opening having a side flap and one or more elastic members to provide an elasticized leg cuff. U.S. Patent 4,909,803 entitled "Disposable Absorbent Article Having Elasticized Flaps" issued to Aziz et al. on March 20, 1990, describes a disposable diaper having "stand-up" elasticized flaps (barrier cuffs) to improve the containment of the leg regions. U. S. Patent 4,695,278 entitled "Absorbent Article Having Dual Cuffs" issued to Lawson on September 22, 1987; and U. S. Patent 4,795,454 entitled "Absorbent Article Having Leakage-Resistant Dual Cuffs" issued to Dragoo on January 3, 1989, describe disposable diapers having dual cuffs including a gasketing cuff and a barrier cuff. U.S. Patent 4,704,115 entitled "Disposable Waist Containment Garment" issued to Buell on November 3, 1987, discloses a disposable diaper or incontinence garment having side-edge-leakage-guard gutters configured to contain free liquids within the garment.

While each elasticized leg cuff 52 may be configured so as to be similar to any of the leg bands, side flaps, barrier cuffs, or elastic cuffs described above, it is preferred that the elasticized leg cuff 52 includes an elastic gasketing cuff 62 with one or more elastic strands 64 as shown in Fig. 2, which is described in the above-referred U.S. Patent Nos. 4,695,278 and 4,795,454. It is also preferred that each elasticized leg cuff 52 further includes inner barrier cuffs 54 each including a barrier flap 56 and a spacing means 58 which are described in the above-referenced U.S. Patent No. 4,909,803.

The pull-on garment 20 preferably further includes an elasticized waistband 50 that provides improved fit and containment. The elasticized waistband 50 is that portion or zone of the pull-on garment 20 which is intended to elastically expand and contract to dynamically fit the wearer's waist. The elasticized waistband 50 preferably extends longitudinally outwardly from the waist edge of the pull-on garment 20 toward the waist edge of the absorbent core 25. Preferably, the pull-on garment 20 has two elasticized waistbands 50, one positioned in the back region 28 and one positioned in the front region 26, although other pull-on garment embodiments can be constructed with a single elasticized waistband. The elasticized waistband 50 may be constructed in a number of different configurations including those described in U.S. Patent 4,515,595 entitled "Disposable Diapers with Elastically Contractible Waistbands" issued to Kievit et al. on May 7, 1985 and the above referenced U.S. Patent 5,151,092 issued to Buell.

The waistbands 50 may include materials that have been "prestrained" or "mechanically prestrained" (i.e., subjected to some degree of localized pattern mechanical stretching to permanently elongate the material). The materials may be prestrained using deep embossing techniques as are known in the art. Alternatively, the materials may be prestrained by directing the material through an incremental mechanical stretching system as described in U.S.

Patent No. 5,330,458 entitled "Absorbent Article With Elastic Feature Having A Portion Mechanically Prestrained" issued to Buell et al., on July 19, 1994. The materials are then allowed to return to their substantially untensioned condition, thus forming a "zero strain" stretch laminate that is extensible, at least up to the point of initial stretching. Examples of "zero strain" laminate are described hereinafter.

At least one pair of the ear panels 46 and 48 includes the elastic member 70 as shown in Fig. 3. The elastic member 70 of the front ear panels 46 includes an elastomeric material layer 124 (not shown in Fig. 3) which preferably extends laterally outward from the chassis 41 to provide good fitness by generating the optimal retention (or sustained) force at the waist and side areas of the wearer. Preferably, the elastomeric material layer 124 is extensible in at least one direction, preferably in the lateral direction to generate a retention (or sustained) force that is optimal to prevent the pull-on garment 20 from drooping, sagging, or sliding down from its position on the torso without causing the red marking on the skin of the wearer. In a preferred embodiment, each of the ear panels 46 and 48 includes the elastomeric material layer 124.

The elastic member 70 is operatively joined to the outer cover nonwoven layer 74 and preferably the nonwoven webs 72 in the ear panels 46 and 48 to form a laminate. In a preferred embodiment, the elastic member 70 is operatively joined to the nonwoven webs 72 and 74 by securing them to at least one, preferably both of the nonwoven webs 72 and 74 while in a substantially untensioned (zero strain) condition.

The elastic member 70 can be operatively joined to the nonwoven webs 72 and 74, by using either an intermittent bonding configuration or a substantially continuous bonding configuration. Herein, "intermittently" bonded laminate web means a laminate web wherein the plies are initially bonded to one another at discrete spaced apart points or a laminate web wherein the plies are substantially unbonded to one another at discrete spaced apart areas. Conversely, a "substantially continuously" bonded laminate web means a laminate web wherein the plies are initially bonded substantially continuously to one another throughout the areas of interface. It is preferred that the stretch laminate be bonded over all or a significant portion of the stretch laminate so that the inelastic webs (i.e., the nonwoven webs 72 and 74) elongate or draw without causing rupture, and the layers of the stretch laminates are preferably bonded in a configuration that maintains all of the layers of the stretch laminate in relatively close adherence to one another after the incremental mechanical stretching operation. Consequently, the elastic panel members and the other plies of the stretch laminate are preferably substantially continuously bonded together using an adhesive. In a particularly preferred embodiment, the adhesive selected is applied with a control coat spray pattern at a basis weight of about 7.0 grams/m$^2$. The adhesive pattern width is about 6.0 cm. The adhesive is preferably an adhesive such as is available from Nitta Findley Co., Ltd., Osaka, Japan, under the designation H2085F. Alternatively, the elastic panel member and any other components of the stretch laminates may be intermittently or continuously bonded to one another using heat bonding, pressure bonding, ultrasonic bonding, dynamic mechanical bonding, or any other method as is known in the art.

After the elastic member 70 is operatively joined to the nonwoven webs 72 and 74, at least a portion of the resultant composite stretch laminate is then subjected to mechanical stretching sufficient to permanently elongate the non-elastic components which are, for example, the nonwoven webs 72 and 74. The composite stretch laminate is then allowed to return to its substantially untensioned condition. At least one pair of, preferably both of the ear panels 46 and 48 is thus formed into "zero strain" stretch laminates. This configuration allows the ear panels 46 and 48 to be elastically extensible in at least the lateral direction.

A "zero strain" stretch laminate is one type of elastic laminate which is preferably used for such disposable products. For example, methods for making "zero strain" stretch laminate webs are disclosed in U.S. Patent No. 5,167,897 issued to Weber et al. on December 1, 1992; U.S. Patent No. 5,156,793 issued to Buell et al. on October 20, 1990; and U.S. Patent No. 5,143,679 issued to Weber et al. on September 1, 1992. In a manufacturing process for such "zero strain" stretch laminate, an elastomeric material is operatively joined to at least one non-elastic component material in a substantially untensioned (zero strain) condition. At least a portion of the resultant non-elastic composite stretch laminate is then subjected to mechanical stretching sufficient to permanently elongate the non-elastic component material. The composite stretch laminate is then allowed to return to its substantially untensioned condition. Thus, the elastic laminate is formed into a "zero strain" stretch laminate. Herein, "zero strain" stretch laminate refers to a laminate comprised of at least two plies of material which are secured to one another along at least a portion of their coextensive surfaces while in a substantially untensioned ("zero strain") condition; one of the plies comprising a material which is stretchable and elastomeric (i.e., will return substantially to its untensioned dimensions after an applied tensile force has been released) and a second ply which is elongatable (but not necessarily elastomeric) so that upon stretching the second ply will be, at least to a degree, permanently elongated so that upon release of the applied tensile forces, it will not fully return to its original undeformed configuration. The resulting stretch laminate is thereby rendered elastically extensible, at least up to the point of initial stretching, in the direction of initial stretching.

The elastic member 70 is preferably joined to, more preferably directly secured to the respective edges 78 of the liquid impervious film (i.e., the liquid impervious film 68) through an adhesive 76 as shown in Fig. 3. In a preferred embodiment, while liquid impervious film 68 longitudinally extends in the front, back and crotch regions 26, 28 and 30, it does not laterally extend into at least one of, preferably each of the extensible ear panels 46 and 48. In a more preferred embodiment, the elastic member 70 is joined to the respective edges 78 of the liquid impervious film 68 at the outer-facing surface 77 as shown in Fig. 3. In an alternative embodiment, the elastic member 70 may be joined to the respective edges 78 of the liquid impervious film 68 at the body-facing surface 79 (not shown in Figs.). Preferably, the adhesive 76 is applied in a spiral glue pattern. In a preferred embodiment, the adhesive 76 is a flexible adhesive with an amorphous and crystallizing component. Such a preferred adhesive is made by Nitta Findley Co., Ltd., Osaka, Japan, under the designation H2085F. Alternatively, the elastic member 70 may be joined to the respective edges 78 of the liquid impervious film 68 by any other bonding means known in the art which include heat bonds, pressure bonds, ultrasonic bonds, dynamic mechanical bonds, or combinations of these attachment means.

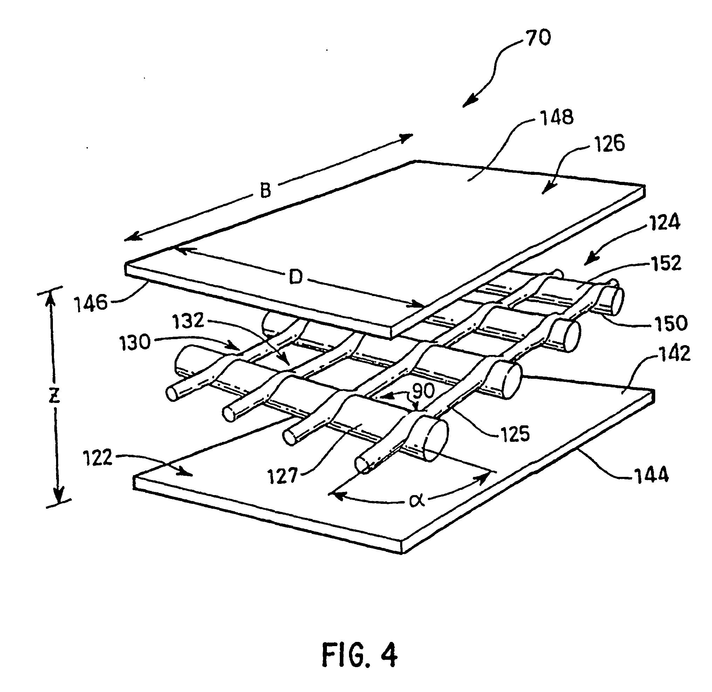

Referring to Fig. 4, the elastic member 70 includes the elastomeric material layer 124 having a first surface 150 and a second surface 152 opposing the first surface 150, and a first coverstock layer 122 which is joined to the first surface 150 of the elastomeric material layer 124. In a preferred embodiment, the first coverstock layer 122 is joined to the first surface 150 of the elastomeric material layer 124 by an adhesive (not shown in Fig. 4). More preferably, the elastic member 70 further includes a second coverstock layer 126 which is joined to the second surface 152 of the elastomeric material layer 124 by an adhesive (not shown in Fig. 4).

Preferably, the elastic member 70 is joined to the respective edges 78 of the liquid impervious film 68 at the outer-facing surface 77 as shown in Fig. 3. In an alternative embodiment, the elastic member 70 may be joined to the respective edges 78 of the liquid impervious film 68 at the body-facing surface 79 (not shown in Figs.).

The elastomeric material layer 124 may be formed in a wide variety of sizes, forms and shapes. In a preferred embodiment, the elastomeric material layer 124 is in the form of a continuous plane layer. Preferred forms of continuous plane layer include a scrim, a perforated (or apertures formed) film, an elastomeric woven or nonwoven, and the like. The continuous plane layer may take any shape which can be suitably provided in the ear panels. Preferred shapes of continuous plane layer include a quadrilateral including a rectangle and a square, a trapezoid, and the other polygons. In an alternative embodiment, the elastomeric material layer 124 is in the form of discrete strands (or strings) which are not connected each other.

Elastomeric materials which have been found to be especially suitable for the elastomeric material layer 124 are styrenic block copolymer based scrim materials, perforated (or apertured) elastic films, preferably with a thickness of from about 0.05 mm to about 1.0 mm (0.002 inch - 0.039 inch). Other suitable elastomeric materials for the elastomeric material layer 124 include "live" synthetic or natural rubber, other synthetic or natural rubber foams, elastomeric films (including heat shrinkable elastomeric films), elastomeric woven or nonwoven webs, elastomeric composites, or the like.

In the preferred embodiment shown in Fig. 4, the elastomeric scrim 124 has a plurality of first strands 125 and a plurality of second strands 127. The plurality of first strands 125 intersect the plurality of second strands 127 at nodes 130 at a predetermined angle $\alpha$, forming a net-like open structure having a plurality of apertures 132. Each aperture 132 is defined by at least two adjacent first strands and at least two adjacent second strands, so that the apertures 132 are substantially rectangular in shape. Other configurations of the apertures 132, such as parallelograms, squares, or circular arc segments, can also be provided. Preferably, the first and second strands 125 and 127 are substantially straight and substantially parallel to one another. Preferably, the first strands 125 intersect the second strands 127 at nodes 130 such that the angle $\alpha$ is about 90 degrees. The first and second strands 125 and 127 are preferably joined or bonded at nodes 90.

A preferred elastomeric scrim 124 is manufactured by the Conwed Plastics Company (Minneapolis, Minn., U.S.A.) under the designation XO2514. This material has about 12 elastic strands per inch in the structural direction B (i.e., the first strands 125) and about 7 elastic strands per inch in the structural direction D (i.e., the second strands 127).

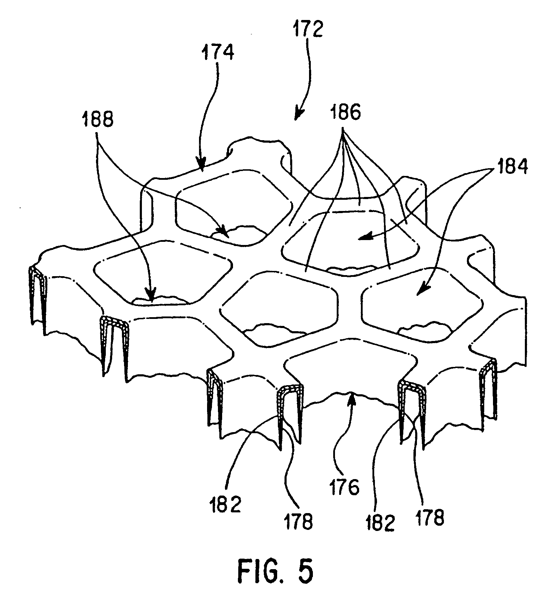

In an alternative preferred embodiment, the elastomeric material layer 124 is a porous, macroscopically-expanded, three-dimensional elastomeric web. Referring to Fig. 5, the porous, macroscopically-expanded, three-dimensional elastomeric web 172 has a continuous first surface 174 and a discontinuous second surface 176 opposing the first surface 174. The elastomeric web 172 preferably comprises a formed film interconnecting member 186 including at least two polymeric layers 178 and 182. The first layer 178 is substantially elastic and the second layer 182 is substantially less elastic than the first layer 178. At least one of the two polymeric layers 178 and 182 is formed from a polystyrene thermoplastic elastomer. The elastomeric web 172 exhibits a multiplicity of primary apertures 184 in the first surface 174 of the web 172. The primary apertures 184 are defined in the plane of the first surface 174 by a continuous network of the interconnecting member 186. The interconnecting member 186 exhibits an upwardly concave-shaped cross-section along its length. The interconnecting member 186 also forms secondary apertures 188 in the plane of the second surface 176 of the web 172. The apertures 184 and 188 may take any shape. A preferred elastomeric web is disclosed in International Publication No. WO 98/3716 (Curro et al.), published on August 27, 1998. A preferred porous elastomeric material for the elastomeric material layer is available from Tredegar Film Products under the designation X-25007.

In the embodiment shown in Fig. 4, the elastic member 70 includes first and second coverstock layers 122 and 126, and elastomeric material layer 124 disposed in the first and second coverstock layers 122 and 126. The first coverstock layer 122 has an inner surface 142 and an outer surface 144. The inner surface 142 of the first coverstock layer 122 is the surface that is positioned facing the elastomeric material layer 124. The second coverstock layer 126 also has an inner surface 146 and an outer surface 148. The inner surface 146 of the second coverstock layer 126 is the surface that is positioned facing the elastomeric material layer 124. The elastomeric material layer 124 also has two planar surfaces, first surface 150 and second surface 152, each of which is substantially parallel with the planes of the first and second coverstock layers 122 and 126. The first surface 150 is that planar surface of the elastomeric material layer 124 that is most closely adjacent with the inner surface 142 of first coverstock layer 122. The second surface 152 is that planar surface of elastomeric material layer 124 that is most closely adjacent to the inner surface 146 of the second coverstock layer 126.

Since the elastic member 70 will be subjected to mechanical stretching before and during use, the first and second coverstock layers 122 and 126 need to have a relatively high elongation at breaking, and are more preferably stretchable or elongatable, yet more preferably drawable (but not necessarily elastomeric), without undue (and preferably without any), tearing or ripping. Further, the first and second coverstock layers 122 and 126 are preferably compliant, soft feeling, and non-irritating to the wearer's skin and give the article the feel and comfort of a cloth garment.

An exemplary preferred nonwoven material for the first and second coverstock layers 122 and 126 is a consolidated nonwoven material available from the Fiberweb North America, Inc. (Simpsonville, South Carolina, U.S.A.) under the designation Sofspan 200. Herein, "consolidated nonwoven material" means a nonwoven material that has been gathered or necked under mechanical tension in the structural direction D so that the material can elongate in the structural direction D under low force. This material has a basis weight of 25 $g/m^2$ before consolidation and a basis weight of about 63g/m2 after consolidation. Alternatively the spunbonded nonwoven of polypropylene/polyethylene copolymer of the present invention may be used for the first and second coverstock layers 122 and 126.

The elastomeric material layer 124 and the first and second coverstock layers 122 and 126 are joined together, preferably by using an adhesive, to form the elastic member 70. A preferred method for making the elastic member 70 is described in International Publication No. WO 98/55298 (Langdon et al.) published on December 10, 1998.

TEST METHODS

The hand value of Koshi and the fuzz level of a nonwoven sample are calculated based on the physical properties which are obtained from the measurements described in the following. The physical properties include 1) Tensile property; 2) Bending property; 3) Surface property; 4) Shearing property; 5) Compression property; and 6) Weight and thickness. These properties include in total sixteen (16) characteristic values or detailed properties as indicated in the Table I.

Table I

| Property | Symbols | Property | Unit | Remarks |
|---|---|---|---|---|
| Tensile | LT | linearity of load-extension curve | none | LT = 1 : completely linear and LT=0 : extremely non-linear |
| | WT | tensile energy per unit area | gf • cm /cm$^2$ | Higher value of WT corresponds to higher extensibility. |
| | RT | tensile resilience | % | RT = 100% : completely elastic RT = 0% : completely inelastic |
| | EM | extensibility | % | Strain at maximum load (= 50 gf/cm) |

Table I   (continued)

| Property | Symbols | Property | Unit | Remarks |
|---|---|---|---|---|
| Bending | B | bending rigidity | gf • cm²/cm | Bending rigidity per unit width of fabric. |
| | 2HB | hysteresis of bending moment | gf • cm/cm | Hysteresis of bending moment observed in the bending moment-curvature relationship. A larger value of 2HB means a greater fabric inelasticity |
| Shearing | G | shear stiffness | gf/cm degree | |
| | 2HG | hysteresis of shear force at 0 degree of shear angle | gf/cm | |
| Compression | LC | linearity of compression-thickness curve | none | LC = 1: completely linear LC = 0: completely non-linear |
| | WC | compressional energy | gf • cm/cm² | A larger value of WC corresponds to higher compressibility. |
| | RC | compressional resilience | % | RC = 100% : elastic and RC = 0% : completely inelastic |
| Surface | MIU | coefficient of friction | none | Higher value corresponds to higher friction. |
| | MMD | mean deviation of MIU | none | Higher value corresponds to larger variation of friction. |
| | SMD | geometrical roughness | μm | Higher value corresponds to geometrically rough surface. |
| Weight and Thickness | W | sample weight | mg/cm² | |
| | $T_o$ | sample thickness | mm | Thickness at pressure of 0.5 gf/cm² |

The sixteen characteristic values in the Table I are obtained by the following measurement and analytical methods.

A. Measurement and Calculation for Hand Value of Koshi

1) Tensile property:

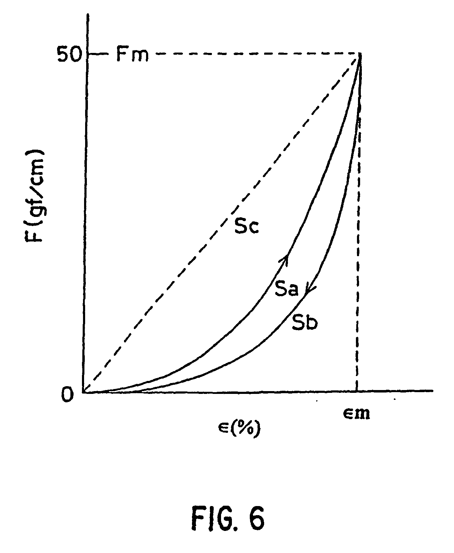

The nonwoven sample is subjected to applied unidirectional extension stress up to a maximum load of 50 gf/cm and then allowed to return to its initial state. The speed of the deformation is 0.1 mm/s. The effective dimension of the sample is 20 cm in width and 2.5 cm in length (rectangular). As a result, the tensile property curve as shown in Fig. 6 is obtained by the measurement. The horizontal axis shows the strain (%) and the vertical axis shows the stress (gf/cm). The characteristic values of LT, WT, and RT are calculated as follows:

$$LT = (Sa + Sb) / (Sa + Sb + Sc) \tag{1}$$

$$WT = Sa + Sb \tag{2}$$

$$RT = Sb / (Sa + Sb) \tag{3}$$

where Sa, Sb, Sc are defined by the areas shown in Fig. 6.

2) Bending property:

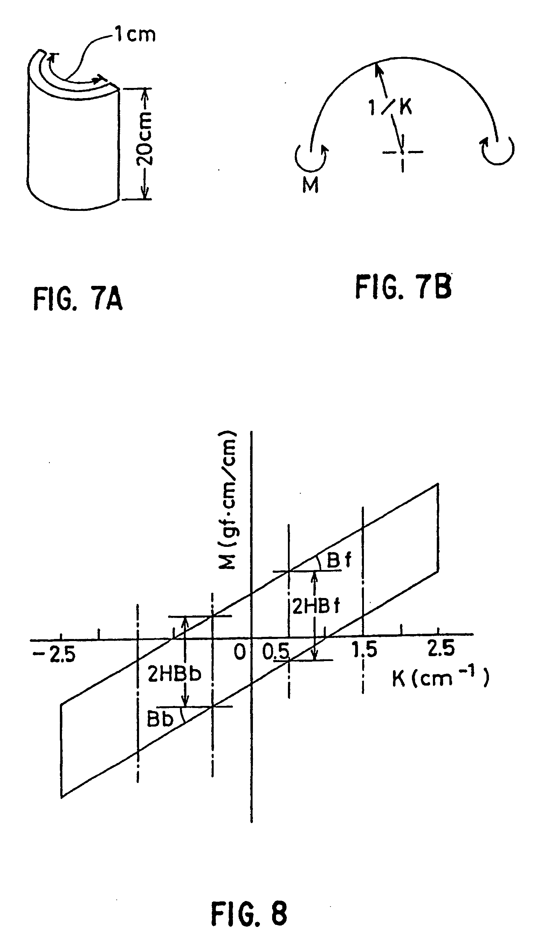

The deformation mode is a pure bending between the curvature K = -2.5 cm$^{-1}$ and 2.5 cm$^{-1}$. It is a measure of the force required to bend the sample. The effective dimension for the measurement is 2.o cm in length and 1.0 cm in width (rectangular). The sample is bent as shown in Figs. 7A and 7B. The bending rate is 0.5 cm$^{-1}$/sec. As a result, the bending hysteresis curve as shown in Fig. 8 is obtained by the measurement. The horizontal axis shows the curvatures K cm$^{-1}$ and the vertical axis shows the moment M (gf·cm/cm). The values of B and 2HB are calculated as follows:

$$B = (Bf + Bb) /2 \tag{4}$$

where Bf and Bb are the slopes of the hysteresis curves between K = 0.5 cm$^{-1}$ and 1.5 cm$^{-1}$ and K = -0.5 cm$^{-1}$ and -1.5 cm$^{-1}$ respectively.

$$2HB = ( 2HBf + 2HBb ) / 2 \tag{5}$$

where 2HBf and 2HBb are the hysteresis differences at K = 0.5 cm$^{-1}$ and -0.5 cm$^{-1}$, respectively.

3) Surface property:

To measure the surface roughness of the sample, a pianowire is prepared and bent as shown in Figs. 7A and 7B. 5.0 gf (allowance, ± 0.5 gf) of the contact force is applied by a spring of which spring constant is 25 ± 1 gf/mm. The natural frequency of the system should be more than 30 Hz when the contactor is out of the contact.

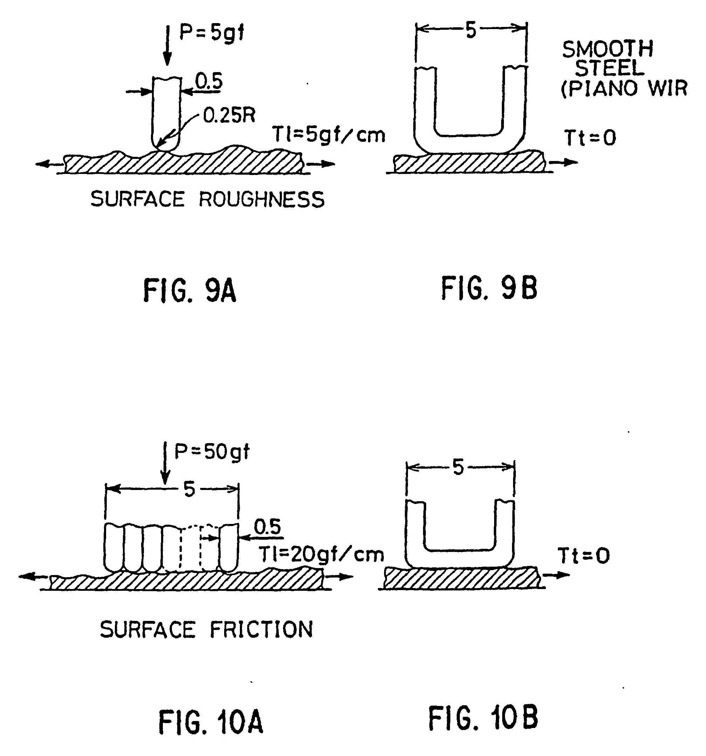

The friction between the surfaces of the sample and a contactor is measured under a constant contact pressure. Surface friction should be measured by using the contactor shown in Fig. 10A and 10B. The surface of the contactor is covered by ten parallel and stacked piano steel wires. The ten pieces of the same wires are placed on the surface of specimen. The compressional force of 50 gf by dead weight is applied to the surface of the sample through the contactor.

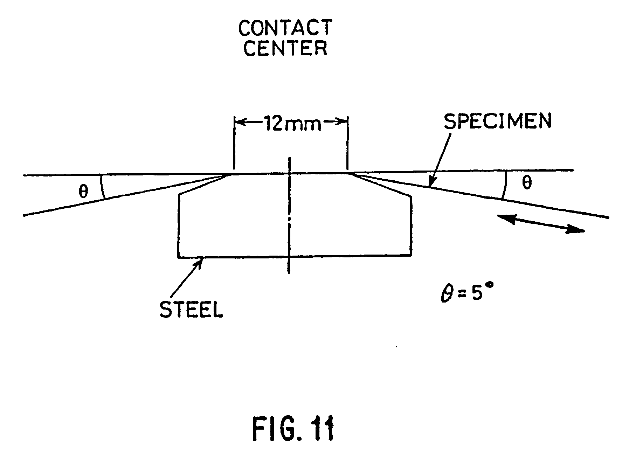

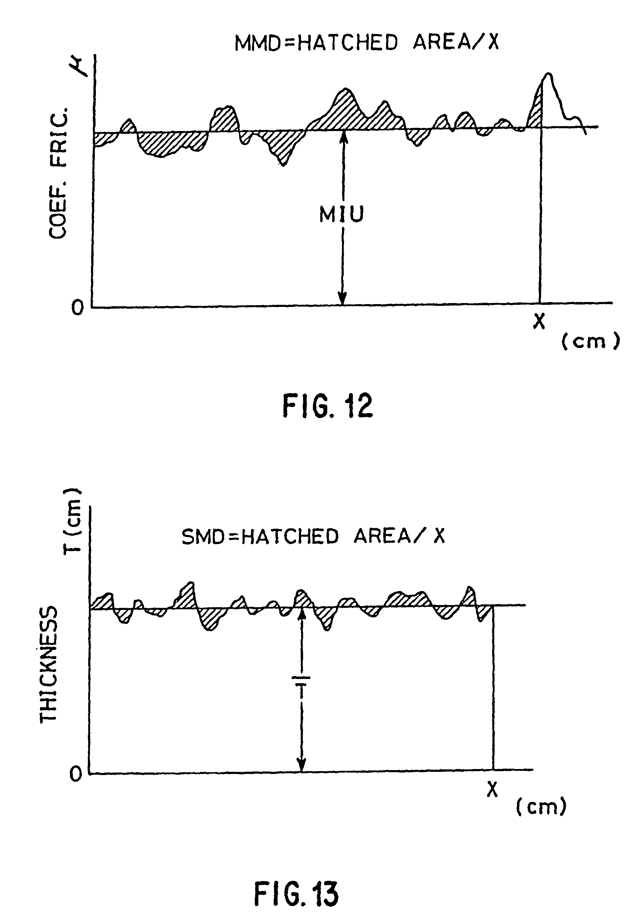

In the both of the roughness and friction measurements, the specimen is moved between 2 cm interval by a constant velocity of 0.1 cm/sec on a smooth steel plate placed horizontally where the tension of the specimen is kept 5.0 gf/cm (force per unit length) and the contactor is kept its position. The dimension of the plate is shown in Fig. 11. thus, the changes of the surface friction coefficient μ and the thickness T are obtained as shown in Figs. 12 and 13.

Consequently, the values of MIU, MMD and SMD are obtained from the following expressions:

$$MIU = \frac{1}{X} \int_{0}^{x} \mu dx, \qquad ----\ (6)$$

$$MIU = \frac{1}{X} \int_{0}^{x} |\mu - \mu'| dx, \qquad ----\ (7)$$

$$SMD = \frac{1}{X} \int_{0}^{x} |T - T'| dx, \qquad ----\ (8)$$

where $\mu$ ; frictional force/compresional force

$\mu'$ ; mean value of $\mu$

x ; displacement of the contactor on the surface of sample

X ; 2 cm is taken in this measurement

T ; Thickness of the sample at position x

T ; Mean value of T

4) Shearing property:

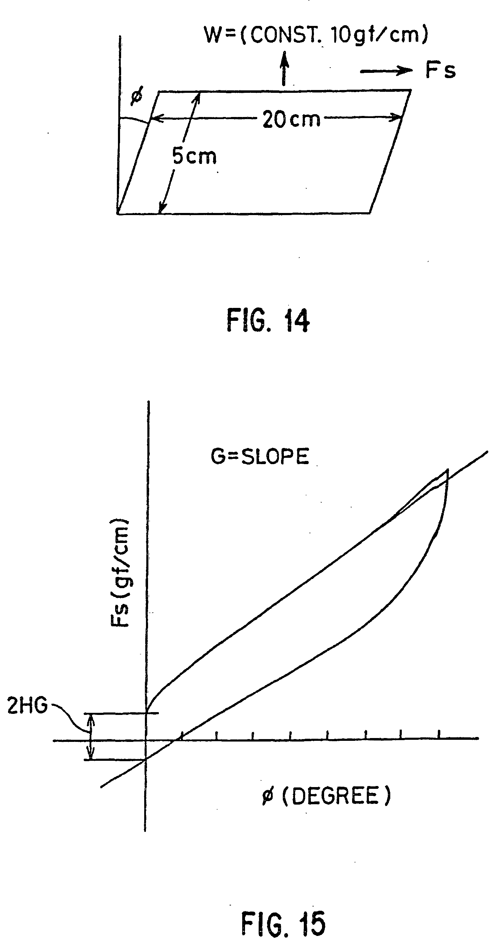

The constant extension force, 5 gf/cm, is applied to the sample uni-directional and then the shear force Fs is superposed in the sample plane along the transverse direction up to the shear angle ø=4° as shown in Fig. 14. Then, the sample shear deformation is recovered by reducing the shear angle back to zero. The effective dimension of the sample is 20 cm in width and 5 cm in length. Thus, the relationship between Fs and ø is obtained as shown in Fig. 15. The value of 2HG is obtained as the hysteresis at ø=0°. The value of G is calculated as follows:

$$G = (Gf + Gb) / 2 \tag{9}$$

where Gf and Gb are the average slopes between ø = 0.5° and 5° and between ø = -0.5° and -5° respectively.

5) Compression property:

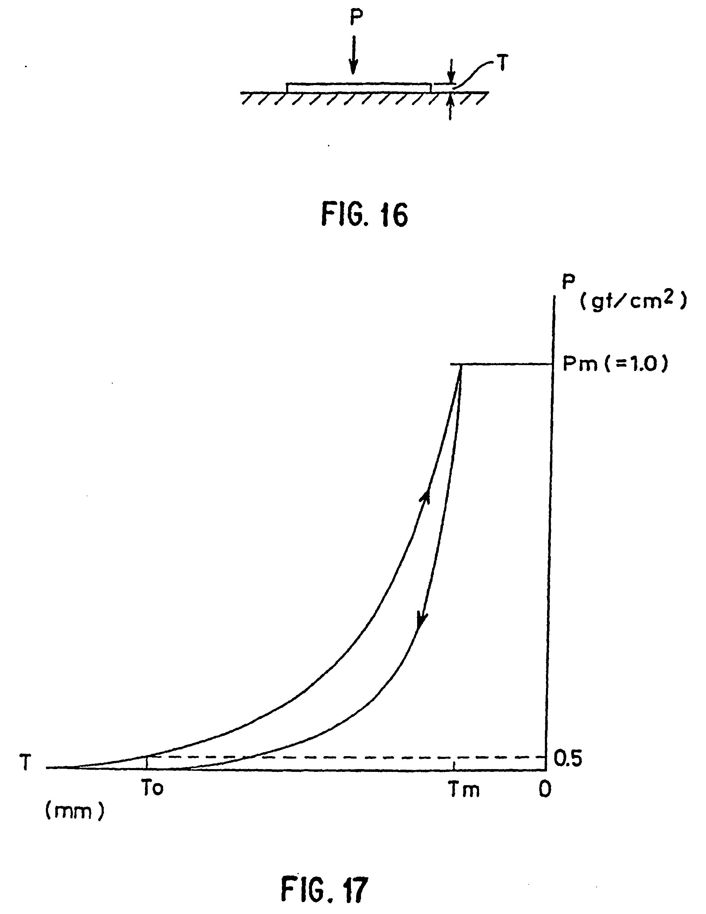

The effective dimension of the sample is 2.5 cm long and 2.0 cm in width is used and the longitudinal direction is taken along either warp or weft direction. 2 cm$^2$ of a circled area is compressed by two circular-plates of steel having 2 cm$^2$ area (Fig. 16). The velocity of the compression is 20 micron/sec and when the pressure attains at 10 g/cm$^2$, the recovery process is measured by the same velocity. The values of LC, WC and RC are obtained by the following expressions:

$$LC = WC / WOC \tag{10}$$

$$WC = \int_{Tm}^{To} PdT \tag{11}$$

$$RC = WC' / WC \tag{12}$$

where

T ; Thickness of the sample (cm).

To ; Thickness of the sample at maximum pressure 0.5 gf/cm$^2$, (cm).

Tm ; Thickness of the sample at maximum pressure Pm which is :

Pm = 50 gf/cm$^2$

WOC; = Pm (To - Tm) / 2 ---- (13)

WC' ; Recovering energy given by the pressure of the recovering process, P' such as

$$WC' = \int_{Tm}^{To} P' \, dT \tag{14}$$

6) Weight and thickness:

The value of T mm is measured as the thickness when the compressional property is measured (P = 0.5 gf/cm$^2$). The value of W g/m$^2$ is measured as the weight per unit area of the sample.

7) Calculation for Hand Values:

The hand value of Koshi is obtained from the following expression (15) by applying the sixteen characteristic values obtained from the measurements. The calculation using the expression (15) is conducted according to the Knit High Sensivity Condition (KN-403-KTV).

$$HV = C_0 + \sum_{16}^{i=1} \{ C_i \cdot (X_i - X_i') / \sigma_i \} \qquad \text{----- (15)}$$

where HV is the hand value.

The numbers and constants to be applied to the equation are indicated in the Tables II and III.

Table II

| Property | i | Xi | Xi' | σi |
|---|---|---|---|---|
| | 0 | | | |
| Tensile | 1 | LT | 0.7756 | 0.0679 |
| | 2 | log WT | 0.6808 | 0.2557 |
| | 3 | log RT | 1.5952 | 0.0639 |
| Bending | 4 | log B | -1.6441 | 0.3288 |
| | 5 | log 2HB | -1.5180 | 0.3213 |
| Shearing | 6 | log 2G | -0.4000 | 0.1276 |
| | 7 | log 2HG | 0.0444 | 0.1486 |
| | 8 | log 2HG5 | 0.0444 | 0.1486 |
| Compression | 9 | LC | 0.6337 | 0.0692 |
| | 10 | log WC | -0.9937 | 0.1526 |
| | 11 | RC | 38.1224 | 5.6815 |
| Surface | 12 | log HIU | -0.5952 | 0.0861 |
| | 13 | log HMD | -1.5999 | 0.2018 |
| | 14 | log SMD | 0.9280 | 0.1999 |
| Weight and Thickness | 15 | log T | 0.0638 | 0.1361 |
| | 16 | W | 17.3383 | 5.0040 |

Table III

| For Koshi | | For Koshi | |
|---|---|---|---|
| i | Ci | i | Ci |
| 0 | 4.4473 | 1 | -0.2437 |
| 6 | 0.9934 | 2 | -0.1740 |
| 7 | -0.0264 | 3 | 0.0931 |
| 8 | 0.4165 | 9 | -0.1255 |
| 4 | 0.5064 | 10 | 0.1252 |
| 5 | 0.3654 | 11 | 0.0119 |
| 15 | -0.1568 | 12 | -0.0125 |
| 16 | 0.2789 | 13 | 0.1037 |

Table III (continued)

| For Koshi | | For Koshi | |
|---|---|---|---|
| i | Ci | i | Ci |
| | | 14 | 0.0276 |

8) Measurement and Analysis Equipment

An example of preferred measurement and analysis equipment is the type KES FB1-FB4 which is available form Kato Tech Co., LTD., Kyoto Japan. The nonwoven sample to be used by this equipment is a square sheet of 20 cm x 20 cm. The measurement and analysis are conducted on at least three (3) samples, more preferably at least ten (10) samples.

B. Fuzz Level Measurement

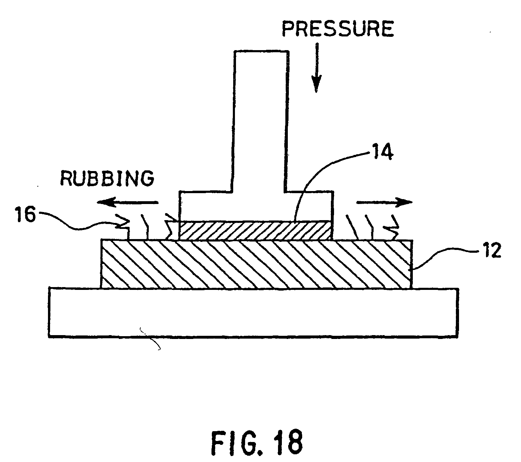

To measure the quantity of untangled fibers that protrude from the surface of the sample, the face of the sample 12 is rubbed against the face of sandpaper 14 for 29 seconds at 0.7 Hz to cut or loose the untangled fibers 16. 2000 gf/cm$^2$ of pressure is applied to the sample 12. An example of the equipment is shown in Fig. 18. The cut fibers produced by this action are collected by a removal tape and quantified with an analytical balance. The fuzz level is defined as the weight of the fibers collected per unit area (mg/cm$^2$).
An example of equipment available is Sutherland Ink Rub Tester. 2000 gf/cm$^2$ of pressure is applied to the sample. This apparatus abrades a 4 cm x I 1 cm piece of sample with a 15 cm x 5.1 cm piece of sandpaper (Matelite K224 Cloth Sandpaper Grit 320-J, Norton Co., Troy, NY). The rub cycle is 20 times at 0.7 cycle/sec. The fibers (fuzz) are removed using two 15 cm x 5.1 cm pieces of removal tape (3M No. 3187 Trans Tape, Cincinnati, OH) from both the sandpaper and sample.
It is understood that the examples and embodiments described herein are for illustrative purpose only and that various modifications or changes will be suggested to one skilled in the art without departing from the scope of the present invention.

**Claims**

1. A disposable diaper (20) comprising a backsheet (22) including an outer cover of a spunbonded nonwoven layer (74), the spunbonded nonwoven layer including component fibers formed by a polypropylene/polyethylene copolymer **characterized in that**
   the component fibers have an average fiber denier of less than 2.5; the copolymer which forms the component fibers is a polypropylene modified by copolymerizing from 0.5% to 20% of polyethylene in the backbone
   the spunbonded nonwoven layer has a Coefficient of Friction against Steel (CFS) of less than 0.29; and
   the spunbonded nonwoven layer has a Fuzz Level (FL) of less than 0.15 mg/cm$^2$.

2. The disposable diaper (20) of Claim 1, wherein the copolymer is a block polypropylene/polyethylene copolymer.

3. The disposable diaper (20) of Claim 2, wherein the copolymer is a random polypropylene/polyethylene copolymer.

4. The disposable diaper (20) of Claim 1, wherein the polypropylene/polyethylene copolymer has a melting point range between 100°C and 180 °C.

5. The disposable diaper (20) of Claim 1, wherein the spunbonded nonwoven layer (74) has an average embossment ratio of from 5% to 25%.

6. The disposable diaper (20) of Claim 1, wherein the spunbonded nonwoven layer (74) has a hand value of Koshi of less than 11.

7. The disposable diaper (20) of Claim 1, wherein the backsheet (22) further includes a liquid impervious layer (68) joined to the body-facing surface of the outer cover fabric (74).

8. The disposable diaper (20) of Claim 7, wherein the liquid impervious layer (68) is a microporous polyethylene film joined to the body-facing surface of the outer cover fabric (74) by an adhesive.

9. The disposable diaper (20) of Claim 1, wherein the component fibers have an average fiber denier of less than 1.9.

**Patentansprüche**

1. Wegwerf-Windel (20) mit einer Außenlage (22), die eine äußere Schicht einer Spinnvlies-Lage (74) umfasst, wobei die Spinnvlies-Lage Komponenten-Fasern umfasst, die durch ein Polypropylen/Polyethylen-Copolymer gebildet sind, **dadurch gekennzeichnet, dass**
   die Komponenten-Fasern ein durchschnittliches Faserdenier von weniger als 2,5 aufweisen, wobei das Copolymer, welches die Komponenten-Fasern bildet, ein Polypropylen ist, das durch Copolymerisierung von 0,5 % bis 20 % Polyethylen in dem Grundgerüst modifiziert ist;
   die Spinnvlies-Lage einen Reibungskoeffizienten auf Stahl (CFS) von weniger als 0,29 aufweist; und
   die Spinnvlies-Lage einen Flaumgrad (Fuzz Level FL) von weniger als 0,15 mg/cm$^2$ aufweist.

2. Wegwerf-Windel (20) nach Anspruch 1, wobei das Copolymer ein Block-Polypropylen/Polyethylen-Copolymer ist.

3. Wegwerf-Windel (20) nach Anspruch 2, wobei das Copolymer ein ungeordnetes Polypropylen/Polyethylen-Copolymer ist.

4. Wegwerf-Windel (20) nach Anspruch 1, wobei das Polypropylen/Polyethylen-Copolymer einen Schmelzpunkt-Bereich zwischen 100° C und 180° C aufweist.

5. Wegwerf-Windel (20) nach Anspruch 1, wobei die Spinnvlies-Lage (74) ein durchschnittliches Präge-Verhältnis von 5 % bis 25 % aufweist.

6. Wegwerf-Windel (20) nach Anspruch 1, wobei die Spinnvlies-Lage (74) einen Koshi-Hand-Wert von weniger als 11 aufweist.

7. Wegwerf-Windel (20) nach Anspruch 1, wobei die Außenlage (22) ferner eine flüssigkeitsundurchlässige Lage (68) aufweist, die mit der Körperzugewandten Oberfläche des äußeren Schicht-Stoffs (74) verbunden ist.

8. Wegwerf-Windel (20) nach Anspruch 7, wobei die flüssigkeitsundurchlässige Lage (68) eine mikroporöse Polyethylen-Folie ist, die mit der Körper-zugewandten Oberfläche des äußeren Schicht-Stoffs (74) durch einen Klebstoff verbunden ist.

9. Wegwerf-Windel (20) nach Anspruch 1, wobei die Komponenten-Fasern ein durchschnittliches Faserdenier von weniger als 1,9 aufweisen.

**Revendications**

1. Couche jetable (20) comportant une feuille de fond (22) comprenant un recouvrement extérieur d'une couche (74) non tissée filée-liée, la couche non tissée filée-liée comprenant des fibres constituantes formées par un copolymère de polypropylène/polyéthylène, **caractérisée en ce que**
   les fibres constituantes ont un denier de fibre moyen inférieur à 2,5 ; le copolymère qui forme les fibres constituantes est un polypropylène modifié par copolymérisation de 5% à 20% de polyéthylène dans la colonne vertébrale ;
   la couche non tissée filée-liée a un coefficient de frottement contre l'acier (CFS) inférieur à 0,29 ;
   et la couche non tissée filée-liée a un niveau de Fuzz (FL) inférieur à 0,15 mg/cm$^2$.

2. Couche jetable (20) selon la revendication 1, dans laquelle le copolymère est un copolymère séquencé polypropylène/polyéthylène.

3. Couche jetable (20) selon la revendication 2, dans laquelle le copolymère est un copolymère aléatoire de polypropylène/polyéthylène.

**4.** Couche jetable (20) selon la revendication 1, dans laquelle le copolymère de polypropylène/polyéthylène a un point de fusion qui se trouve dans la plage de 100°C à 180°C.

**5.** Couche jetable (20) selon la revendication 1, dans laquelle la couche (74) non tissée filée-liée a un rapport de gaufrage moyen de 5% à 25%.

**6.** Couche jetable (20) selon la revendication 1, dans laquelle la couche (74) non tissée filée-liée a une valeur de maniement de Koshi inférieure à 11.

**7.** Couche jetable (20) selon la revendication 1, dans laquelle la feuille de fond (22) comprend, en outre, une couche (68) imperméable aux liquides réunie à la surface faisant face au corps du tissu de recouvrement extérieur (74).

**8.** Couche jetable (20) selon la revendication 1, dans laquelle la couche (68) imperméable aux liquides est un film microporeux de polyéthylène réuni à la surface faisant face au corps du tissu de recouvrement extérieur (74) par un adhésif.

**9.** Couche jetable (20) selon la revendication 1, dans laquelle les fibres constituantes ont un denier de fibre moyen inférieur à 1,9.

FIG. 1

FIG. 2

FIG. 3

FIG. 4

FIG. 5

FIG. 6

FIG. 7A

FIG. 7B

FIG. 8

P = 5gf

0.5
0.25R

Tl = 5gf/cm

SURFACE ROUGHNESS

**FIG. 9A**

5

SMOOTH STEEL (PIANO WIR

Tt = 0

**FIG. 9B**

P = 50gf

5

0.5
Tl = 20gf/cm

SURFACE FRICTION

**FIG. 10A**

5

Tt = 0

**FIG. 10B**

FIG. 11

MMD=HATCHED AREA/X

FIG. 12

SMD=HATCHED AREA/ X

FIG. 13

29

W=(CONST. 10gf/cm)

$\phi$

20cm

5cm

Fs

**FIG. 14**

G=SLOPE

Fs (gf/cm)

2HG

$\phi$ (DEGREE)

**FIG. 15**

FIG. 16

FIG. 17

PRESSURE

RUBBING

16

14

12

# FIG. 18